# EUROPÄISCHE PATENTSCHRIFT

(11) **EP 2 772 275 B1**
(45) Veröffentlichungstag und Bekanntmachung des Hinweises auf die Patenterteilung: **27.07.2016**
(21) Anmeldenummer: 14156854.3
(22) Anmeldetag: 26.02.2014
(51) Int. Cl.: A61M 1/16, A61B 5/021, A61M 1/36, G05B 13/02

(54) **Fuzzy-Logik**
Fuzzy logic
Logique floue

(30) Priorität: 28.02.2013 DE 102013101989
(43) Veröffentlichungstag der Anmeldung: 03.09.2014
(73) Patentinhaber: B. Braun Avitum AG, 34212 Melsungen (DE)
(72) Erfinder: Atallah, Richard, 34212 Melsungen (DE); Strohhöfer, Christof, Dr., 34123 Kassel (DE); Wagner, Jürgen, Prof. Dr., 34131 Kassel (DE)
(74) Vertreter: Winter, Brandl, Fürniss, Hübner, Röss, Kaiser, Polte - Partnerschaft mbB

(56) Entgegenhaltungen:
- EP-A1- 1 900 384
- EP-A2- 0 661 065
- WO-A1-00/66197
- DE-A1-102008 010 531
- US-A1- 2009 112 102
- NORDIO M ET AL: "PROJECTION AND SIMULATION RESULTS OF AN ADAPTIVE FUZZY CONTROL MOLULE FOR BLOOD PRESSURE AND BLOOK VOLUME DURING HEMODIALYSIS", ASAIO JOURNAL, LIPPINCOTT WILLIAMS & WILKINS / ASAIO, HAGERSTOWN, MD, US, Bd. 40, Nr. 3, 1. Juli 1994 (1994-07-01), Seiten 686-690, XP000498259, ISSN: 1058-2916

## Beschreibung

Die Erfindung betrifft ein System und ein Verfahren, das bei einer Blutbehandlung, z.B. einer Hämodialyse, Hämofiltration oder Hämodiafiltration, einsetzbar ist.

Intradialytische Hypotensive Episoden, IHE, gehören zu den am häufigsten auftretenden Komplikationen während einer Dialysebehandlung. Intradialytische Hypotension kann oft durch schnellen Flüssigkeitsentzug oder falsch bestimmtes Trockengewicht ausgelöst werden. Eine typische Ursache kann eine starke Reduzierung des zentralen Blutvolumens und eine inadäquate Antwort auf diese Verringerung sein. Zahlreiche Algorithmen und Methoden zur Vermeidung dieser Hypotensionen wurden bisher entwickelt. Jedoch konnten intradialytische Hypotensive Episoden nicht komplett vermieden werden.

Aus der EP 0 956 872 A2 ist ein System eines blutdruckbasierten physiologischen Regelkreises mit einem Fuzzy-Controller bekannt, der Blutdruckeingangswerte in Echtzeit evaluiert und die Ultrafiltrations (UF)-Rate entsprechend stellt.

Die EP 0 661 065 A2 offenbart ein Infusionssystem mit einer Regeleinrichtung, die eine Medikamentendosierung automatisch an den von mehreren Faktoren beeinflussten Patientenzustand anpasst. Das Infusionssystem erfasst dabei mehrere qualitativ unterschiedliche Einflusskomponenten als unscharfes Wissen und wertet diese aus. Die ermittelten Messgrößen werden von einer zentralen Messwertverarbeitungseinheit erfasst. Von dieser werden weitere Größen abgeleitet.

WO 00/66197 A1 offenbart ein Hämofiltationssystem sowie eine Methode, um flüssige oder lösliche Schadstoffe aus dem Blut eines Patienten zu entfernen. Das System überwacht kontinuierlich die Flussrate von Blut, abgeleiteter Flüssigkeit und Infusionsflüssigkeit. Eine Überwachungseinrichtung überwacht Parameter des Patienten wie beispielsweise Herzrate und Blutdruck und justiert die Pumpenraten dementsprechend. Der Überwachungscontroller (Supervisory Controller) benutzt Fuzzy-Logic Steuerung, um Entscheidungen zu treffen. Diese basieren auf einem Satz von Überwachungsregeln, um jede Pumpenrate zu kontrollieren und eine gewünschte Flussrate zu erreichen, und um auf den Fehlerfall reagieren zu können. Der Überwachungscontroller besteht aus vier separaten Fuzzysystemen, wobei nur eines der Fuzzysysteme zu einer bestimmten Zeit aktiv sein kann.

In dem Artikel "Projection and simulation results of an adaptive Fuzzy Control Module for blood pressure and blood volume during hemodialysis" aus dem ASAIO Journal vom 1.7.1994, Bd. 40, Nr. 3, Seiten 686-690, XP000498259 wird ein Fuzzy-Logic-basiertes Verfahren offenbart, mit dem man das Verhalten des Blutdrucks eines Patienten während einer Dialysesitzung kontrollieren kann. Die Aufgabe ist es hier, mit einer Dialysesitzung das Patientetrockengewicht zu erreichen, und das innerhalb einer bestimmten Zeit, wobei der systolische Blutdruck des Patienten nicht mehr als 10 Prozent unter den Startwert fallen darf

Wenn zur Vermeidung von intradialytischen Morbiditäten durch Einbeziehen eines physiologischen Regelkreises ein hämodynamischer Parameter, z.B. entweder der Blutdruck, BD, oder das relative Blutvolumen, RBV, geregelt wird, können nachteilige Effekte auftreten. Ein Nachteil der Regelung des Blutdrucks ist das Unwohlbefinden des Patienten durch die hohe Anzahl an Blutdruck-Messungen mit Manschette. Die Reduzierung der Anzahfvon BlutdruckMessungen pro Therapie löst zwar das Problem der häufigen Blutdruck-Messungen, ruft aber ein anderes Problem hervor, da der Patienten-Blutdruck über längere Zeiträume unüberwacht bleibt. Das relative Blutvolumen kann mit Hilfe eines externen oder in der Dialysemaschine verbauten Sensors (z.B. Hämatokrit-Sensor) ohne Verlust von Patientenkomfort in sehr kurzen Zeitabständen (< 1s) gemessen werden. Die Regelung des relativen Blutvolumens ist zwar kontinuierlich, zeigt aber keine Korrelation mit dem pre-, post- und intradialytischen Blutdruck. Andere Untersuchungen ergaben, dass mit der Regelung des relativen Blutvolumens eine bis zu 30%-ige Reduzierung von hypotensiven Episoden erreichbar ist.

Ein Ziel dieser Erfindung ist es, blutbehandlungsbedingte, z.B. intradialytische Morbiditäten, unter anderem intradialytische hypotensive Episoden, zu reduzieren.

Mit der Erfindung wird ein System gemäß dem Patentanspruch 1 oder einem oder mehreren der System-Unteransprüche geschaffen. Weiterhin wird ein Verfahren gemäß dem nebengeordneten Verfahrensanspruch oder einem oder mehreren der Verfahrensunteransprüche bereitgestellt.

Bei einem, mehreren oder allen Ausführungsbeispielen ist ein neu entwickelter physiologischer Regelkreis vorgesehen, der mindestens zwei physiologische Parameter des Patientenzustands evaluiert, gewichtet und sie mit Hilfe eines physiologischen Regelkreises regelt. Hierbei wird bei einem, mehreren oder allen Ausführungsbeispielen die UF-Rate als Stellgröße entsprechend gestellt. Die zwei konkreten physiologischen Parameter können z.B. der Blutdruck und das relative Blutvolumen sein. Allerdings können alternativ oder zusätzlich auch andere relevante Größen, z.B. die Sauerstoffsättigung des Bluts, die Herzrate, etc. ebenfalls mit in die Regelung einbezogen werden.

Bei einem, mehreren oder allen Ausführungsbeispielen werden blutbehandlungsbedingte, z.B. intradialytische Morbiditäten, unter anderem intradialytische hypotensive Episoden, durch einen Experten-physiologischen Regelkreis vermieden oder zumindest reduziert.

Gemäß einem Aspekt der Erfindung ist ein Verfahren oder System zur Blutbehandlung bereitgestellt, das dazu ausgelegt ist, mindestens zwei hämodynamische Parameter, beispielsweise Blutdruck und relatives Blutvolumen, während der Blutbehandlung zu erfassen, wobei mindestens zwei Fuzzy-Module, die als Eingangsgrößen Messwerte der hämodynamischen Parameter aufnehmen, mindestens ein Gewichtungsmodul, dem mindestens zwei der von den Fuzzy-Modulen abgegebenen Ausgangssignale zuführbar sind, und mindestens eine Einstelleinrichtung zur Einstellung mindestens einer Stellgröße, beispielsweise einer Ultrafiltrationsrate, UFR oder UF-Rate, einer Dialysierflüssigkeitsleitfähigkeit oder einer Dialysierflüssigkeitstemperatur, in Abhängigkeit von einem von dem Gewichtungsmodul abgegebenen Ausgangssignal vorhanden sind.

Das Verfahren oder System kann zur Erfassung oder Vermeidung von intradialytischen hypotensiven Episoden ausgelegt sein, wobei die überwachten hämodynamischen Parameter zwei oder mehr der folgenden Größen umfassen können:
Blutdruck, Blutdruckverlauf, relatives Blutvolumen, relativer Blutvolumenverlauf, Hämatokritwert, Hämatokritverlauf, Sauerstoffsättigung, Sauerstoffsättigungsverlauf des Bluts, Herzrate, Herzratenverlauf
Absorbanz oder Absorbanzverlauf urämischer Toxine oder ähnliche hämodynamische Parameter, oder
weitere physikalische Parameter wie etwa von dem System gemessene Blutdruckwerte wie beispielsweise arterieller und/oder venöser Blutdruck und/oder ihre Verläufe.

Das System kann als Dialysegerät für eine Hämodialyse, Hämofiltration oder Hämodiafiltration ausgelegt sein. Das Verfahren kann dabei gleichfalls für eine Hämodialyse, Hämofiltration oder Hämodiafiltration ausgelegt sein.

Das Verfahren oder System kann dazu ausgelegt sein, die Blutdruckwerte diskontinuierlich in bestimmten Zeitintervallen zu messen, mit einem Grenzwert zu vergleichen und bei Abfallen des Blutdruckwerts unter den Grenzwert auf kontinuierliche Blutdruckmessung überzugehen.

Es kann z.B. ein Kurzzeit-Fuzzy-Modul, STFM, vorhanden sein, das dazu ausgelegt ist, das Verhalten des Blutdrucks in einem zurückliegenden Zeitraum zu bewerten und anhand von Evaluierungsregeln eine den Zustand des Patienten widerspiegelnde Variable zu berechnen.

Alternativ oder zusätzlich kann ein Langzeit-Fuzzy-Modul, LTFM, vorgesehen sein, durch das der Verlauf des Blutdrucks über einen längeren Zeitraum ausgewertet wird, der länger sein kann als der von dem vorgenannten Fuzzy-Modul (Kurzzeit-Fuzzy-Modul) ausgewertete Zeitraum.

Bei dem Verfahren oder System kann eine Messeinheit in Form eines Hämosensors, und ein weiteres Fuzzy-Modul zur Auswertung des von dem Hämosensor abgegebenen Messsignals vorgesehen sein, wobei z.B. das Blutvolumen sowie der Blutvolumenverlauf eines Patienten ausgewertet werden können. Der Hämosensor kann z.B. ein Hämatokritsensor sein, oder auch ein Hämoglobinsensor oder ein Sauerstoffsättigungssensor, oder eine andere Form eines einen oder mehrere Blutparameter erfassenden Sensors sein. Der Hämosensor kann z.B. einen oder mehrere der folgenden hämodynamischen Parameter erfassen:
Blutdruck, Blutdruckverlauf, relatives Blutvolumen, relativer Blutvolumenverlauf, Hämatokritwert, Hämatokritverlauf, Sauerstoffsättigung, Sauerstoffsättigungsverlauf des Bluts, Herzrate, Herzratenverlauf, Absorbanz urämischer Toxine oder ähnliche hämodynamische Parameter oder hämodynamische Verläufe, oder weitere physikalische Parameter oder physikalische Verläufe, wie etwa von dem System gemessene Blutdruckwerte wie beispielsweise arterieller und/oder venöser Blutdruck bzw. arterieller und/oder venöser Blutdruckverlauf. Diese Sensoren werden als Hämosensoren bezeichnet.

Optional können mindestens zwei oder drei der Fuzzy-Module jeweils einen eine Hypotonierelevanz, im Folgenden Hyporelevanz genannt, darstellenden Ausgangswert (hre1, hre2, hre3) bilden, wobei ein Gewichtungsmodul dazu ausgelegt sein kann, die von den Fuzzy-Modulen gebildeten Hyporelevanz-Ausgangswerte zu einem resultierenden Hyporelevanz-Ausgangswert zu kombinieren. Ein weiteres Fuzzy-Modul kann z.B. den resultierenden Hyporelevanz-Ausgangswert zusammen mit einem relativen Ultrafiltrationsvolumen, das das Verhältnis zwischen dem aktuellen und dem gesamten Ultrafiltrationsvolumen beschreibt, evaluieren und eine entsprechende Ultrafiltrations-Sollrate berechnen.

Bei einem, mehreren oder allen Ausführungsbeispielen können mindestens zwei oder drei der Fuzzy-Module jeweils einen eine Hyporelevanz darstellenden Ausgangswert bilden, wobei das Gewichtungsmodul dazu ausgelegt ist, die von den Fuzzy-Modulen gebildeten Hyporelevanz-Ausgangswerte zu einem resultierenden Hyporelevanz-Ausgangswert zu kombinieren, und mit einem weiteren Fuzzy-Modul, das den resultierenden Hyporelevanz-Ausgangswert zusammen mit einem relativen Ultrafiltrationsvolumen, das das Verhältnis zwischen dem aktuellen und dem gesamten Ultrafiltrationsvolumen beschreibt, oder die relative Zeit, die das Verhältnis aus der aktuellen und der gesamten Zeit beschreibt, evaluiert, und eine entsprechende Ultrafiltrations-Sollrate berechnet.

Bei dem Verfahren oder System kann der relative Blutvolumenverlauf, RBV, in Form einer RBV-Kurve gespeichert werden, die RBV-Kurve mit unterschiedlichen Fenstergrößen mit einem Algorithmus, beispielsweise einem Algorithmus kleinster Quadrate angenähert werden, und der Blutvolumenverlauf in Zeitabständen überwacht werden, die einer Fenstergröße entsprechen, bei der sich eine ausreichend gute Überlappung von beispielsweise 50% oder mehr in dem tatsächlichen Blutvolumenverlauf ergibt.

Die Erfindung wird nachstehend anhand von Ausführungsbeispielen unter Bezugnahme auf die Zeichnungen näher beschrieben.
Fig. 1 zeigt ein erfindungsgemäßes Ausführungsbeispiel eines physiologischen Regelkreises,
Fig. 2 zeigt ein Ausführungsbeispiel eines Dialysegeräts,
Fig. 3 zeigt Schaubilder für eine linear least square fit-Annäherung des Blutdrucks,
Fig. 4 veranschaulicht Fuzzy-Sets von Eingangsvariablen und einer Ausgangsvariable eines LTFM,
Fig. 5 illustriert gefittete RBV-Kurven mit unterschiedlichen Fenstergrößen,
Fig. 6 zeigt ein Aktivitätsdiagramm zum Anpassen des RBVs in z.B. 10 Minuten Intervallen,
Fig. 7 zeigt einen Median des extrapolierten RBVs und Mittelwert der UFR bei z.B. 292 Dialysetherapien,
Fig. 8 bis 11 zeigen Fuzzy-Sets für verschiedene Beispiele von RBV-Mustern bzw. Hyporelevanz,
Fig. 12 zeigt ein weiteres Ausführungsbeispiel des erfindungsgemäßen Systems,
Fig. 13 gibt ein Ablaufdiagramm der Blöcke Eins und Zwei des Systems an,
Fig. 14 zeigt Simulationsergebnisse von Block Eins,
Fig. 15 veranschaulicht Simulationsergebnisse von Block Zwei,
Fig. 16 zeigt ein Ablaufdiagramm von Block Drei,
Fig. 17 illustriert Simulationsergebnisse von Block Drei,
Fig. 18 zeigt Simulationsergebnisse der Kombination von Block Eins und Zwei
Fig. 19 zeigt ein Ausführungsbeispiel eines erfindungsgemäßen Verfahrens in Form eines Ablaufdiagramms des kompletten physiologischen Regelkreises, und
Fig. 20 zeigt Simulationsergebnisse des ganzen Systems.

Bei einem, mehreren oder allen Ausführungsbeispielen ist ein physiologischer Regelkreis vorgesehen, der mehrere physiologische Parameter, die den Zustand des Patienten während seiner Dialysebehandlung widerspiegeln, überwacht. Anhand der Erkenntnisse, die aus diesen Parametern gewonnen werden, werden bei einem, mehreren oder allen Ausführungsbeispielen Intradialytische Morbiditäten, unter anderem hypotensive Episoden, vorzeitig erkannt. Ihre Verhinderung bzw. Beseitigung wird durch Anpassung von Stellgrößen erreicht, die dazu beitragen, hypotensive Episoden rechtzeitig zu verhindern.

Als Beispiel dieser physiologischen Parameter sind bei einem, mehreren oder allen Ausführungsbeispielen der Erfindung der Blutdruck (BD) und das relative Blutvolumen (RBV) in Betracht genommen. Die Stellgröße ist zum Beispiel in Form der Ultrafiltrationsrate (UF-Rate) realisiert.

Der physiologische Regelkreis besteht bei einem, mehreren oder allen Ausführungsbeispielen aus einer oder mehreren, z.B. zwei, Messeinheiten, und einer oder mehreren, z.B. fünf, Evaluierungseinheiten. Eine erste Messeinheit ist bei einem, mehreren oder allen Ausführungsbeispielen ein Blutdruck-Messmodul, das den Blutdruck, also Blutdruckwerte, im Folgenden auch als BD oder BD-Werte bezeichnet, in unterschiedlichen Zeitintervallen überwacht. Die zweite Messeinheit ist bei einem, mehreren oder allen Ausführungsbeispielen ein Hämatokritsensor, der das relative Blutvolumen des Patienten kontinuierlich überwacht. Eine oder zwei der Evaluierungseinheiten evaluieren das Verhalten des Blutdrucks, wo jede Einheit einen Wert ausgibt, der den Patientenzustand beschreibt. Eine z.B. dritte Evaluierungseinheit evaluiert das Verhalten des relativen Blutvolumens und berechnet ebenso einen Wert, der den Patientenzustand beschreibt. Diese berechneten Werte werden bei einem, mehreren oder allen Ausführungsbeispielen in einer vierten Evaluierungseinheit gewichtet, die die drei Zustandswerte zu einem resultierenden Wert kombiniert. Dieser resultierende Wert wird durch eine fünfte Evaluierungseinheit ausgewertet, die eine entsprechende UF-Rate berechnet.

Alle Evaluierungseinheiten, oder alle außer einer, können Fuzzy-Module sein, die linguistische Variablen (Eingangsvariablen) evaluieren. Die eine verbleibende, nicht als Fuzzy-Modul ausgelegte Evaluierungseinheit kann z.B. eine Gewichtungseinheit sein, welche die Zustandswerte zu einem resultierenden Wert zusammenführt.

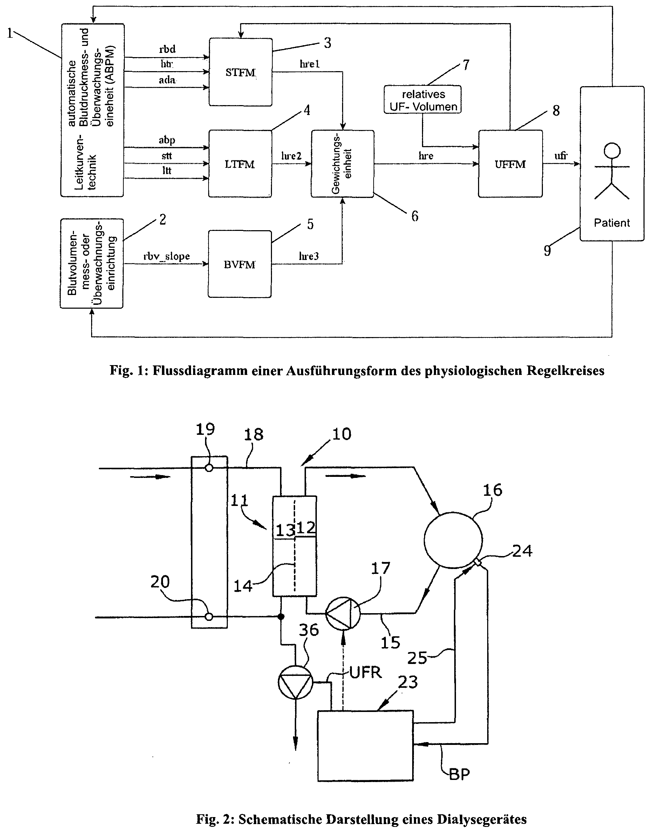

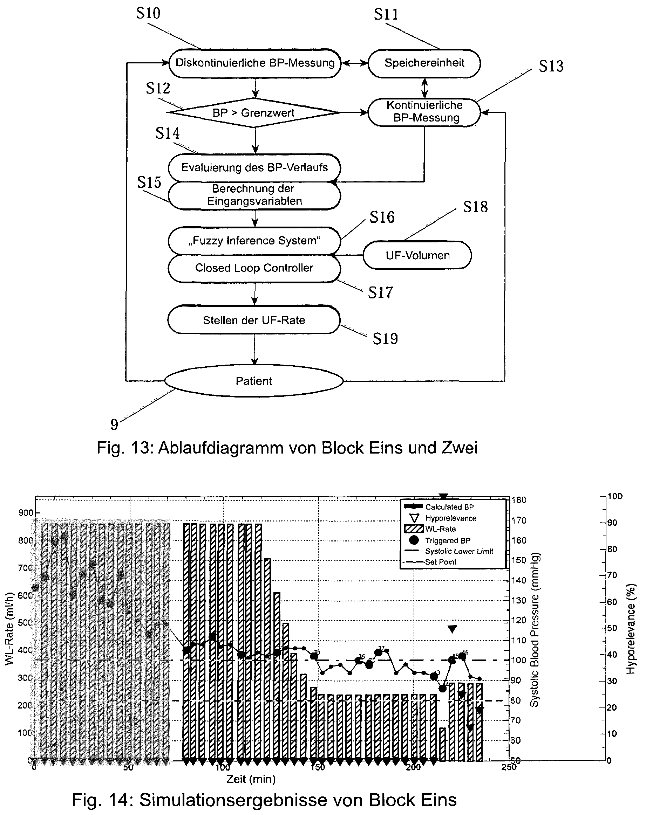

Fig. 1 stellt ein Schaltbild eines Ausführungsbeispiels des physiologischen Regelkreises dar. Wie oben beschrieben, liefert eine automatische Blutdruckmess- und - überwachungseinrichtung ("Automatic Blood Pressure Measurement, ABPM") 1, gegebenenfalls zusammen mit einer z.B. in der EP 1226838A2 beschriebenen Leitkurven-Technik, auch GuideLine Technik genannt, Blutdruckwerte, hier auch als BD oder BD-Werte bezeichnet, aus einer Speichereinheit, die beispielsweise eine interne oder externe Festplatte des automatischen Blutdruck-Erfassungs-Moduls 1 oder des Systems sein kann, kontinuierlich, z.B. in Intervallen von beispielsweise 5 Minuten (oder länger oder kürzer). Diese Werte bestehen aus berechneten und aktuell gemessenen BD-Werte, die diskontinuierlich oder kontinuierlich gemessen werden können. Eine kontinuierliche Messung wird durchgeführt, sobald der aktuell gemessene BD-Wert unter einer vordefinierten Grenze fällt. Die kontinuierliche Blutdruckmessung wird weiter durchgeführt, solange der aktuell gemessene Blutdruck unter einer vordefinierten Grenze bleibt. Ein erstes Fuzzy-Modul 3 in Form eines Kurzzeit- Fuzzy-Moduls (Short Time Fuzzy Module, STFM), empfängt von der Einrichtung 1 drei Eingangsvariablen, rbd, htr und ada, die aus den gemessenen und berechneten BD-Werten stammen. Anhand dieser Variablen wird in einem Fuzzy Inference System (s. Fig. 13) das Verhalten des Blutdrucks in dem zurückliegenden Zeitraum, z.B. in den letzten 30 Minuten, evaluiert. Das STFM 3 besteht aus z.B. 48 Regeln, die ausgewertet werden, um hieraus eine Variable zu berechnen, die den Zustand des Patienten widerspiegelt. Diese Variable wird als Hyporelevanz eins (hre1) bezeichnet.

Ein zweites Fuzzy-Modul 4, das ein Langzeit-Fuzzymodul, Long Time Fuzzy Module LTFM, ist, erhält wiederum von der Einrichtung (ABPM) 1 und ggf. der Speichereinheit z.B. drei Eingangsvariablen bpd, stt und Itt, die nur aus den gemessenen BD-Werten bestehen. Diese Variablen bpd, stt und Itt evaluieren das Verhalten des Blutdrucks innerhalb z.B. der letzten 120 Minuten z.B. in einem Fuzzy Inference System. Die Variable bpd ist eine BD-Differenz aus z.B. den zwei letztgemessenen BD-Werten. Die Variable stt ist die Differenz z.B. des letzten und des drittletzten mit einem linearen fit gefitteten BD-Wertes. Die Variable Itt ist die Differenz z.B. des letzten und des fünftletzten mit einem linearen fit gefitteten BD-Wertes. Diese drei Variablen werden im LTFM durch die Fuzzy-Sets und ihre entsprechende Regeln in einem Fuzzy Inference System evaluiert (s. Fig. 13). Dabei wird eine neue Zustandsvariable berechnet, die Hyporelevanz zwei (hre2).

Da das Aufblasen der BD-Manschette eine hohe Belastung für den Patienten darstellt, kann der Blutdruck nicht in kürzeren Zeitabständen überwacht werden. Deshalb wird eine sogenannte GuideLine Technik eingesetzt, bei der der Blutdruck aus patienteneigenen gespeicherten BD-Verläufen berechnet und in der aktuellen Therapie appliziert wird.

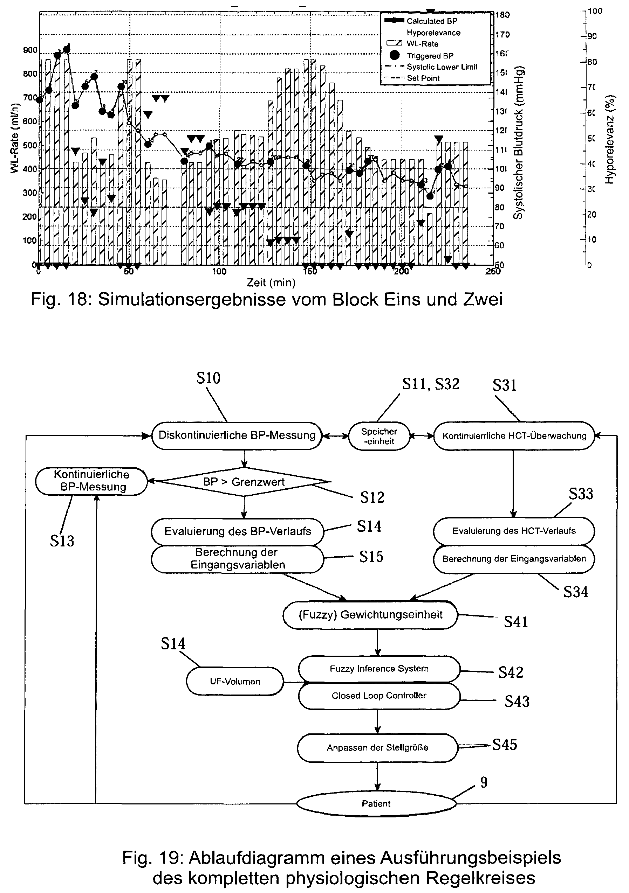

Wie bei jedem Vorhersagesystem besteht eine Ungenauigkeit in den berechneten Vorhersagepunkten, in diesem Fall in den berechneten BD-Werten. Diese Ungenauigkeit kann durch eine zweite Messeinheit, den Hämatokritsensor, verringert werden. Aus diesem Grund wird ein drittes Fuzzy-Modul 5 eingesetzt, das Blutvolumen Fuzzy-Modul, BVFM, das seine Eingangsgröße von einer das relative Blutvolumen erfassenden Blutvolumen-Mess- oder überwachungseinrichtung 2 empfängt und den relativen Blutvolumenverlauf des Patienten 9 interpretiert. In Intervallen von z.B. 10 Minuten bei einer 50 %-igen Überlappung wird die Blutvolumenkurve, deren Koordinaten in der Speichereinheit gespeichert werden, in Echtzeit z.B. in einem Fuzzy Inference System evaluiert (s. Fig. 19). Diese Evaluation wird in Form einer dritten Zustandsvariable, der Hyporelevanz drei (hre3) ausgedrückt.

Die Mess- und Überwachungseinrichtungen 1, 2 sind mit einem Patienten 9 verbunden, wie dies in Fig. 1 dargestellt ist.

Die drei durch die drei Fuzzy-Module gebildeten Hyporelevanzen hre1, 2, 3 werden in einer Gewichtungseinheit 6 zu einer resultierenden Hyporelevanz (hre) kombiniert.

Die endgültige, einen drohenden, zu niedrigen Blutdruck angebende Hyporelevanz hre wird zusammen mit dem von einem Block 7 stammenden relativen UF-Volumen, das das Verhältnis des aktuellen und des gesamten UF-Volumen beschreibt, in einem vierten Fuzzy-Modul (UFFM) 8 evaluiert, das an dieser Stelle eine entsprechende UF-Rate berechnet.

Bei einem, mehreren oder allen Ausführungsbeispielen der Erfindung wird ein physiologischer Regelkreis mit mindestens zwei Regelgrößen eingesetzt. Bei der Blutbehandlung, z.B. der Dialyse, werden bei dem physiologischen Regelkreis zwei oder mehr Patientenparameter als Regelgrößen angewendet, z.B. der Blutdruck und das Relative Blutvolumen. Hierbei werden zwei oder mehr Größen aus zwei oder mehr unterschiedlichen Sensoren geregelt. Bei dem dargestellten Ausführungsbeispiel des physiologischen Regelkreises werden zwei hämodynamische Parameter, der Blutdruck und das relative Blutvolumen geregelt.

Der physiologische Regelkreis ist bei einem, mehreren oder allen Ausführungsbeispielen modular aufgebaut, und weist bei dem dargestellten Ausführungsbeispiel fünf Module auf: drei Eingangsmodule 3 bis 5, ein Zwischenmodul 6 und ein Ausgangsmodul 8. Die drei Eingangsmodule sind die BD-Fuzzy-Module 3, 4 (LTFM und STFM), die das Lang- und Kurzzeitverhalten des BDs evaluieren, und das Blutvolumen-Fuzzy-Modul 5 (BVFM), das den RBV-Verlauf des Patienten 9 evaluiert. Jedes dieser drei Module 3 bis 5 berechnet eine Evaluierungsvariable, die Informationen über das Verhalten der hämodynamischen Regelgrößen, die den Zustand des Patienten widerspiegeln, liefert.

In dem vierten Modul 6, dem Gewichtungsmodul, werden die drei Evaluierungsvariablen, die aus den ersten drei Eingangsmodulen 3 bis 5 entstanden sind, zu einer endgültigen Evaluierungsvariable hre kombiniert.

Das fünfte Modul 8 bewertet hauptsächlich zusammen mit einer Größe, die Informationen über das restliche UF-Volumen liefert, die endgültige Evaluierungsvariable, wobei die UF-Rate berechnet wird.

Bei einem, mehreren oder allen Ausführungsbeispielen kann eine indirekte Gewichtung der Regelgrößen erfolgen.

Aus Patientenkomfort-Gründen sollte der Blutdruck nicht in kürzeren Zeitabständen gemessen werden. Während der Zeit, in der keine BD-Werte gemessen werden, sorgt die GuideLine Technik dafür, berechnete BD-Werte, die aus patienteneigenen BD-Werten aus vergangenen Therapien stammen, auf die aktuelle Therapie anzuwenden. Wie bei jedem Vorhersagesystem besteht eine Ungenauigkeit in den berechneten Vorhersagepunkten, in diesem Fall in den berechneten BD-Werten. Diese Ungenauigkeit kann durch die zweite Messeinheit, den Hämatokritsensor, verringert werden. In der Zeit, während der die BD-Werte berechnet werden, werden die Informationen aus dem Hämatokritsensor und aus den berechneten BD-Werten zu unterschiedlichen Prozentanteilen gewichtet, sodass aussageschwache Informationen durch geringere Gewichtung abgewertet und aussagekräftige Informationen durch hohe Gewichtung aufgewertet werden.

Durch den modularen Aufbau ist der physiologische Regelkreis beliebig ausbaubar, so dass eine leichte Einbeziehung von weiteren physiologischen Parametern möglich ist. Es können Eingangsmodule für beliebige Messgrößen hinzugefügt werden. Jedes dieser Eingangsmodule gibt eine Hyporelevanz aus. Diese Hyporelevanzen werden in dem Zwischenmodul 6, entweder auf Basis von vorgegebenen Bedingungen oder auf Basis von entsprechenden Fuzzy-Sets, zu einer Gesamthyporelevanz kombiniert. Auf dieser Gesamthyporelevanz basiert dann die Einstellung der Stellgröße, die in dem Fuzzy-Modul 8 realisiert ist. Darüber hinaus ist auch die Verwendung verschiedener Stellgrößen möglich. Entweder werden diese auf Basis der vom einzigen Zwischenmodul 6 berechneten Hyporelevanz gestellt, oder es werden verschiedene Hyporelevanzen durch mehrere Zwischenmodule berechnet, die dann zur Einstellung je einer Stellgröße verwendet werden.

Aufgrund dieses modularen Aufbaus dieses physiologischen Regelkreises können weitere hämodynamische Parameter in das System als unabhängige Module einbezogen werden. Diese können z.B. die Sauerstoffsättigung, die Herzrate, die Absorbanz urämischer Toxine oder ähnliche hämodynamische Parameter sein. Als Stellgrößen können zusätzlich zu, oder anstelle, der UF-Rate die Dialysierflüssigkeitstemperatur, die -leitfähigkeit oder ähnliche Parameter herangezogen werden.

Neben den hämodynamischen Parametern können auch weitere physikalische Parameter, wie z.B. die an der Maschine gemessenen BD-Werte wie der arterielle und der venöse Blutdruck einbezogen werden.

Aufgrund der Überwachung und Regelung von Patientenparametern durch mehrere hämodynamische Sensoren, welche Informationen über die Kreislaufstabilität des Patienten liefern, kann eine hohes Patienten-Wohlbefinden während der Dialyse erreicht werden. Dies schlägt sich zum einen in einer Verringerung der hypotensiven Episoden während der Dialyse nieder, zum anderen in erhöhtem Patientenkomfort durch eine Reduktion von BD-Messungen mit Manschette.

Es besteht bei einem oder mehreren Ausführungsbeispielen auch die Möglichkeit einer kontinuierlichen Blutdruckmessung. In einem solchen Fall kann ein Fuzzy-Modul entfallen, z.B. das Modul 4.

Der physiologische Regelkreis ist bei einem, mehreren oder allen Ausführungsbeispielen modular aufgebaut. Es ist also ein modulares System mit Einzel- und Gesamtfunktion geschaffen. Dadurch können einzelne Module nach Wunsch aus- und eingeschaltet werden, je nachdem welche Inputparameter und welche Sensoren zur Verfügung stehen und welche Größen-Regelungen vom Arzt gewünscht sind. In das System können problemlos weitere Patientenparameter modular einbezogen werden, ohne die Funktionalität anderer Module zu beeinflussen.

Durch die Gewichtung einzelner Informationen wird eine Abwertung weniger aussagekräftiger Informationen und eine Aufwertung aussagekräftigerer Informationen erreicht. Die Gewichtung wertet Aussagen mit geringer Aussagekraft ab und Aussagen mit hoher Aussagekraft auf.

Im Folgenden werden die einzelnen Module, die in Fig. 1 dargestellt sind, näher beschrieben.

Das STFM-Modul 3 und das UFFM-Modul 8 sind in der EP 0 956 872 B1 zum Teil beschrieben. In dem hier vorliegenden physiologischen Regelkreis ist das UFFM-Modul 8 zum Teil modifiziert.

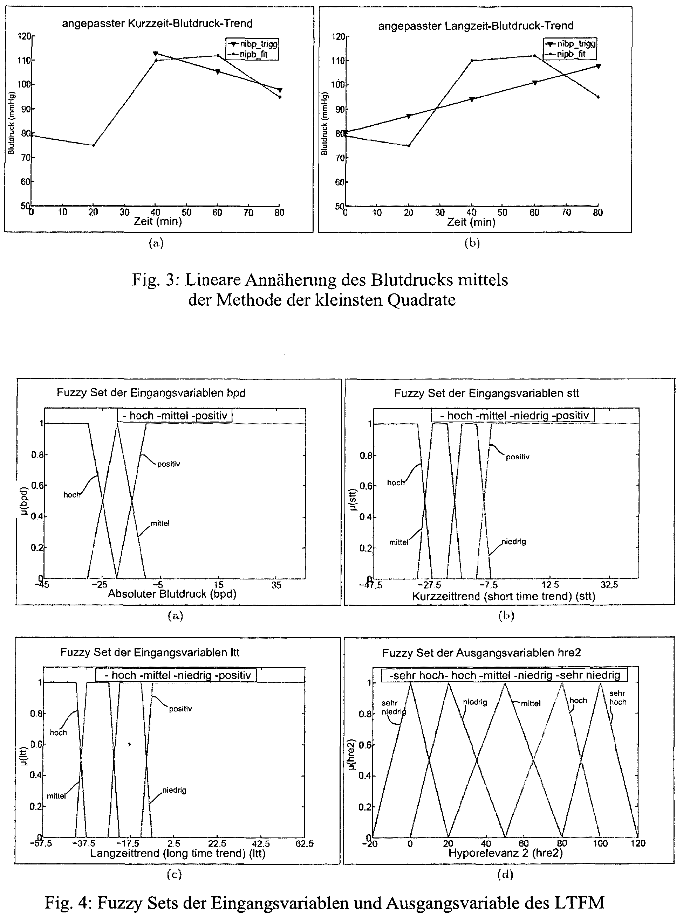

Das LTFM-Modul 4 ist ein von den anderen Modulen unabhängiges Fuzzy-Modul, das aus dem ABPM-Modul 1 drei Eingangsvariablen, die nur aus den gemessenen BD-Werten berechnet werden, erhält. Diese sind die Blutdruckdifferenz (bpd), der Kurzzeittrend, Short Time Trend (stt) und der Langzeittrend, Long Time Trend (Itt), die das Verhalten des Blutdrucks innerhalb des letzten Zeitraums von z.B. 120 Minuten evaluieren. bpd ist die BD-Differenz aus den zwei letztgemessenen BD-Werten (bpd = BPᵢ-BPᵢ₋₁). stt ist die Differenz des letzten und des drittletzten mit einem linearen fit gefitteten BD-Wertes (stt = BP_{fit(i)}-BP_{fit(i-2)}). Itt ist die Differenz des letzten und des fünftletzten mit einem linearen fit gefitteten BD-Wertes (Itt = BP_{fit(i)} -BP_{fit(i-4)}). Das Fitten der BD-Werte, das zur Berechnung der Eingangsvariablen stt und Itt notwendig ist, ist in der Fig. 3 dargestellt. stt und Itt werden aus den neuen entstandenen Blutdrücken auf der Fitting-Linie berechnet. Fig. 3 zeigt den linear least square fit des Blutdrucks.

In Fig. 3 ist in der linken Hälfte (Kurve a) der angepasste Kurzzeit-Blutdruck-Trend dargestellt. Die Trigger-Kurve ist mit dreieckförmigen Markierungen dargestellt, während die Messkurve mit runden Punkten wiedergegeben ist. In der rechten Hälfte der Fig. 3 (Kurve b) ist der angepasste Langzeit-Blutdruck-Trend dargestellt. Die hierbei gewonnenen BD-Werte sind ebenfalls durch geradlinige Abschnitte miteinander verbunden.

Die drei vorstehend angegebenen Variablen werden im LTFM-Modul 4 durch die Fuzzy-Sets und ihre entsprechende Regeln evaluiert (siehe Fig. 4). Jeder Eingangsvariable sind verschiedene linguistische Terme zugeordnet (µ(bpd) = {high, middle, positive}, µ(stt) = {high, middle, slight, positive}, µ(ltt) = {high, middle, slight, positive}). Die Anzahl dieser linguistischen Variablen ermöglicht den Aufbau von 3*4*4 = 48 unterschiedlichen Regeln, die wiederum alle möglichen Kombinationen des Verhaltens dieser Variablen bieten. Nach Expertenwissen und statistischen Methoden über die Verteilung dieser Variablen über mehrere Dialysepatienten wurden Bereiche gebildet, in denen sich diese Variablen befinden dürfen. Die Tabelle 2 verschafft einen Überblick über die Eigenschaften der Eingangsvariablen. Tabelle 1 zeigt die Charakteristik des LTFM - Moduls 4.

**Tabelle 1 Charakteristik des LTFM**

| Long Time Fuzzy Module LTFM 4 | | | | | | |
|---|---|---|---|---|---|---|
| Eingangsvariable | stt | | | | | |
| Linguistische Ausdrücke | hoch | mittel | leicht | | positiv | |
| Bereiche (ranges) | [-45 45] | [-30 -15] | [-20 -5] | | [-10 45] | |
| Funktionen | trapezoid | trapezoid | trapezoid | | trapezoid | |
| Eingangsvariable | ltt | | | | | |
| Linguistische Terme | hoch | mittel | leicht | | positiv | |
| Bereiche (ranges) | [-60 -40] | [-45 -25] | [-30 -10] | | [-15 60] | |
| Funktionen | trapezoid | trapezoid | trapezoid | | trapezoid | |
| Eingangsvariable | bpd | | | | | |
| Linguistische Terme | hoch | | mittel | | positiv | |
| Bereiche (ranges) | [-45 -15] | | [-25-10] | | [15 45] | |
| Funktionen | trapezoid | | dreieck | | trapezoid | |
| Ausgangsvariable | hre2 | | | | | |
| Linguistische Terme | sehr niedrig | niedrig (low) | mittel | hoch | | sehr hoch |
| Bereiche (ranges) | [-20 20] | [0 50] | [20 80] | [50 100] | | [80 120] |
| Funktionen | dreieck | dreieck | dreieck | dreieck | | dreieck |

Nach der Evaluierung dieser Variablen im LTFM-Modul 4 wird eine neue Zustandsvariable berechnet, die Hyporelevanz zwei (hre2). Die Fuzzy-Sets und die Bereiche dieser Zustandsvariable wurden so gewählt, dass auf kleine kritische Änderungen des Blutdrucks eine Veränderung der UF-Rate stattfindet, unter Berücksichtigung, dass das Erreichen des UF-Volumens im Fokus bleibt. Die Fuzzy-Sets von hre2 sind in der Fig. 4 dargestellt. Ihre Eigenschaften sind in der Tabelle 2 beschrieben. Fig. 4 zeigt die Fuzzy-Sets der Eingangsvariablen und Ausgangsvariable des LTFM-Moduls 4.

Das BVFM-Modul 5 ist wiederum ein Fuzzy-Modul, in dem ein Fuzzy-Algorithmus implementiert ist, der den Verlauf des relativen Blutvolumens evaluiert. Um den Blutvolumenverlauf des Patienten während seiner Therapie interpretieren zu können, wurden eine hohe Anzahl an Dialysetherapien betrachtet, in denen die Patienten frei von hypotensiven Episoden waren. In diesen Therapien wurde das relative Blutvolumen entsprechend bewertet, ausgehend von der Hypothese, dass das RBV keine Unauffälligkeit bei einer Therapie mit stabilem Patientenzustand aufweist.

Die medizinische Expertise empfiehlt einen intradialytischen Blutvolumenabfall von 5% pro Stunde. Es müssen aber temporäre Fluktuationen in Betracht gezogen werden, um Rückschlüsse auf den Patientenzustand zu ziehen. Dafür muss zuerst eine RBV-Grenze definiert werden. Um dies zu erreichen, wurde eine hohe Anzahl an intradialytischen Blutvolumenverläufen analysiert. Diese wurden aus Therapien gesammelt, die mit einem definierten UF-Profil gefahren worden sind. Die Patienten wurden dabei mit hohen UF-Raten am Anfang der Dialyse und mit niedrigen UF-Raten gegen Ende der Therapie dialysiert.

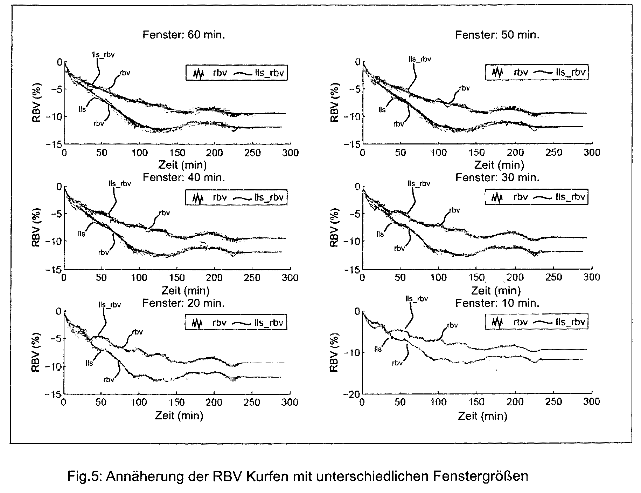

Um temporäre Fluktuationen zu betrachten, muss man die RBV-Kurve in kleine Abschnitte zerlegen. Es ist hierbei zu definieren, wie klein die Abschnitte gewählt werden sollten, um die RBV-Kurve gut reproduzierbar zu machen, ohne wichtige Informationen aus der Kurve zu verlieren. Dafür wurde die RBV-Kurve mit unterschiedlichen Fenstergrößen mit einem linear least square (IIs) Algorithmus gefittet. Die Fig. 5 zeigt ein Beispiel des fits über 6 Fenstergrößen (10, 20, 30, 40, 50 und 60 Minuten) von zwei Blutvolumenkurven. Die sich rasch ändernden Kurven in jedem Fensterplot sind Blutvolumenverläufe zweier Patienten während einer Dialysebehandlung. Die dickeren, sich langsamer ändernden Linien sind die entsprechenden fits mit einem IIs-Algorithmus.

Fig. 5 zeigt gefittete RBV-Kurven mit unterschiedlichen Fenstergrößen mit einer Breite von 10, 20, 30, 40, 50 und 60 Minuten. Fig. 5 zeigt, dass die beste Fenstergröße, die die Kurve am besten fittet, das rechts unten dargestellte Fenster mit dem 10 Minuten Intervall bei einer 50 % Überlappung ist. Durch diese Überlappung kann man den Blutvolumenverlauf in kurzen Zeitabständen überwachen, ohne wichtige Informationen über das Verhalten des Blutvolumens zu verlieren. Nach dem Fitting kann der Verlauf dann bis zu einer Stunde extrapoliert und die Differenz RBVₜ₊₆₀ und RBVₜ gebildet werden (s. die Linien als Beispiel in der Fig. 5).

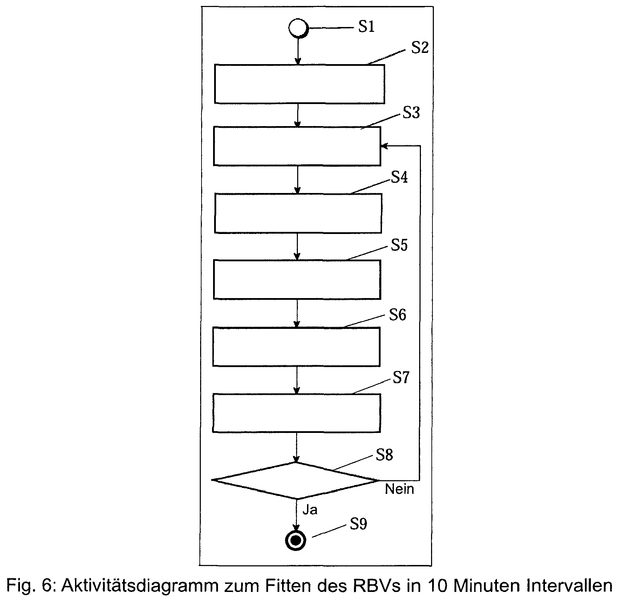

Es sollen als nächstes Grenzen für das Blutvolumen definiert werden. Da RBV-Kurven von stabilen Patienten untersucht wurden, kann man diese als Referenz für stabile RBV-Kurven nehmen und dementsprechend die Grenzen setzen. Um zu untersuchen, in welchen Bereichen das Blutvolumen bei einer gesunden Patientenpopulation schwankt, wurde das in der Fig. 6 beschriebene Aktivitätsdiagramm umgesetzt. Fig. 6 zeigt ein Aktivitätsdiagramm zum Fitten des relativen Blutvolumens in 10 Minuten Intervallen.

Bei dem in Fig. 6 dargestellten Aktivitätendiagramm ist mit einem Schritt S1 der erste Schritt des Anpassungsverfahrens (Fitten) repräsentiert, dem ein Schritt S2 nachfolgt, bei dem Werte des relativen Blutvolumens, die für dieses Beispiel. in 292 Therapiesitzungen gewonnen wurden, in Feldern (Arrays) entsprechend zugeordnet oder untereinander gespeichert werden. Bei einem Schritt S3 werden die RBV-Werte innerhalb der nächsten 10 Minuten bzw. alle 10 Minuten von allen aufgebauten Feldern (Arrays) herausgegriffen. In dem nachfolgenden Schritt S4 werden die herausgegriffenen RBV-Werte in 10-Minutenintervallen mittels eines IIs-Algorithmus ("linear least square", lineare Methode der kleinsten Quadrate) angepasst.

In einem Schritt S5 wird nachfolgend das angepasste relative Blutvolumen auf eine Stunde extrapoliert. In einem Schritt S6 wird dann die Differenz des ersten und des letzten Punkts auf der extrapolierten Linie berechnet, wonach in einem Schritt S7 der Medianwert (Median) von allen berechneten Differenzen berechnet wird. In einem Schritt S8 wird anschließend überprüft, ob alle RBV-Werte verarbeitet worden sind. Wenn dies der Fall ist, endet das Programm bei einem Endpunkt S9. Andernfalls kehrt das Programm zu dem Schritt S3 zurück, so dass die Schritte S3 bis S8 erneut durchlaufen werden.

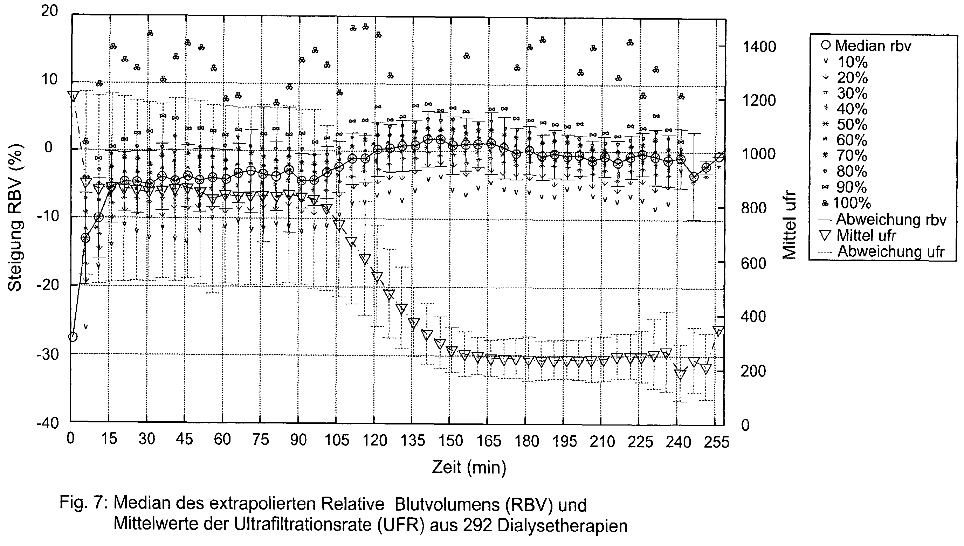

Führt man die Schritte gemäß Fig. 6 durch, so erhält man den in der Fig. 7 dargestellten Medianverlauf der RBV-Werte über die Therapie. Fig. 7 zeigt jeweils den Median des extrapolierten RBV-Wertes und den Mittelwert der UF-Rate bei 292 Dialysetherapien. Die Mittelwerte der UF-Raten an den Fit-Intervallen der RBV-Werte wurden berechnet. Diese sind die Dreiecke in der Fig. 7. Die Sterne spiegeln die prozentigen Zugehörigkeiten des zur Bildung des Medians sortierten RBV-Vectors wider (Median ist die 50%-ige Stelle in diesem Vector). Die Fehlerbalken um die als Kreise angezeigten Medianwerte sind die mittleren absoluten Abweichungen des Medians. Die Fehlerbalken um die als Dreiecke angezeigten Mittelwerte der UF-Raten stellen die Standardabweichungen der UF-Raten dar. Die hohe Standardabweichung bei den UF-Raten wird verursacht durch die unterschiedlichen maximalen UF-Raten bei unterschiedlichen Patienten.

Wie die Fig. 7 zeigt, sind drei Muster zu erkennen. Das erste ist der Medianverlauf von Minute 15 bis Minute 100. Das zweite Muster ist der Medianverlauf von Minute 100 bis Minute 140. Das dritte und letzte Muster ist der Medianverlauf von Minute 140 bis zum Ende der Therapie. Aus diesem Grund sollten dynamische Grenzen gesetzt werden.

Man kann hypothetisch festlegen, dass die Patienten bei einem Median von -4% keine Unauffälligkeiten im Blutvolumenverlauf aufweisen. Dies kann als Grenze gesetzt werden. Alle RBV-Abfälle, die unter dieser Grenze liegen, tendieren zu einem auffälligen Verhalten des RBV-Verlaufs. Um diese Grenze zu tolerieren, wurde die untere Grenze der mittleren absoluten Abweichung in Betracht gezogen. Da diese auch musterabhängig ist, wurde sie bei den unterschiedlichen Mustern unterschiedlich bestimmt. Bei dem ersten und dritten Muster wurde der Mittelwert über die mittlere untere absolute Abweichung gebildet. Diese beträgt -9 % bei dem ersten Muster und -4 % beim dritten Muster. Die Grenzen beim zweiten Muster sind dynamische Grenzen und weisen einen linearen Verlauf auf. Aus diesem Grund wurden beim zweiten Muster dynamische Grenzen von -10 % bis -4 % gebildet.

Diese Grenzen wurden in den Fuzzy-Sets gespiegelt. Es wurden insgesamt zwei linguistische Terme für das Verhalten des relativen Blutvolumens definiert und zwar, µ(rbv) = {critical, normal}. Die Eigenschaften dieser Sets sind in der nachfolgenden Tabelle 2 beschrieben.

**Tabelle 2 Eigenschaften des BVFM**

| Blutvolumen - Fuzzy Modul - BVFF 5 | | | |
|---|---|---|---|
| Eingangsvariable | | rbv-Steigung | |
| Linguistische Terme | | kritisch | normal |
| Bereiche Ranges | Muster 1 | [-20 -4] | [-14 20] |
| | Muster 2 | [-20...0] | [-14...20] |
| | Muster 3 | [-20 0] | [-8 20] |
| Funktion | | trapezoid | trapezoid |
| Ausgangsvariable | | hre3 | |
| Linguistische Terme | | hoch | niedrig |
| Bereiche | | [0 0,8] | [0,2 1] |
| Funktion | | dreieck | dreieck |

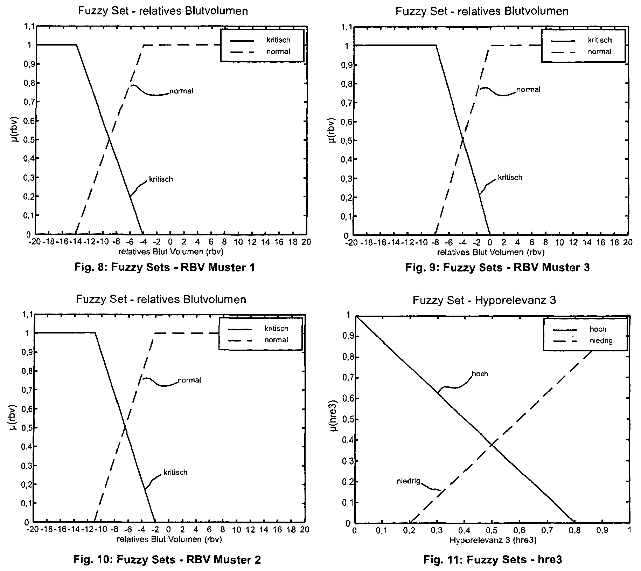

Der relative Blutvolumenabfall wird im BVFM-Modul 5 nach den beschriebenen Fuzzy-Sets und Fuzzy-Regeln evaluiert (s. Fig. 8, Fig. 9, Fig. 10). Als Evaluierungsgröße wird, wie bei den anderen Fuzzy-Modulen, eine dritte Zustandsgröße (hre3) berechnet, deren Fuzzy-Sets und Eigenschaften in der Fig. 11 und in der Tabelle 2 dargestellt sind.

Fig. 8 zeigt Fuzzy-Sets des RBV-Musters 1. Fig. 9 zeigt eins der Fuzzy-Sets des RBV-Musters 3. Fig. 10 zeigt die Fuzzy-Sets des RBV-Musters 2. Fig. 11 zeigt die Fuzzy-Sets des hre3.

Wie aus den Figuren 8 bis 11 ersichtlich ist, ist mit den von 0 auf 1 schräg, linear ansteigenden Kurvenverläufen ein normaler Verlauf dargestellt. Die von 1 auf 0 schräg nach rechts abfallenden Kurven stellen demgegenüber kritische Verläufe dar, anhand derer die in Fig. 11 dargestellte Hyporelevanz hre3 gebildet wird. Auch hier symbolisiert der von 0 auf 1 rechts nach oben ansteigende Kurvenzug einen unkritischen Bereich mit geringer Hyporelevanz, wohingegen der von 1 auf 0 rechts nach unten abfallende Kurvenzug hohe Relevanz bedeutet, d.h. einen kritisch absinkenden Blutdruck repräsentiert.

In der Gewichtungseinheit 6 findet die Evaluierung der einzelnen berechneten Zustandsgrößen, hre1, hre2 und hre3, statt. Die Evaluierung ist eine Form von Kombination aller Zustandsgrößen. Die Gewichtungseinheit kann ein weiteres Fuzzy-Modul sein, in dem die Eingangsvariablen zu linguistischen Termen und Zugehörigkeiten zugeordnet werden. Die Kombination kann aber auch durch skalare Gewichtung der einzelnen Zustandsgrößen geschehen.

Dabei sollten Bedingungen festgelegt werden, welche die Art eines Blutdruckes (getriggert oder berechnet), seine Aussagekraft, die durch die Zustandsgrößen gespiegelt ist, und die Aussagekraft der aus dem Hämatokritsensor gewonnenen Informationen berücksichtigen.

Weitere Möglichkeiten der Erweiterung des Systems bestehen darin, dass ein oder mehrere weitere Eingangs- und/oder Ausgangs-Parameter mit einbezogen werden. Diese können die Sauerstoffsättigung, die Herzrate, und/oder die mit der Maschine gemessenen Drücke, wie z.B. der arterielle oder venöse Blutdruck sein. Diese Eingangsparameter sind Regelparameter und können in unabhängigen Modulen eingeordnet werden. Die Aussagen der einzelnen Eingangsvariablen können unabhängig in Gewichtungseinheiten evaluiert werden. Als Stellgrößen können z.B. neben der UF-Rate die Dialysierflüssigkeitsleitfähigkeit (LF) und die Dialysierflüssigkeitstemperatur (DT) in unabhängigen Fuzzy-Modulen, dem Dialysierflüssigkeitsleitfähigkeits-Fuzzy-Modul (DLFM) und dem Dialysierflüssigkeitstemperatur-Fuzzy-Modul (DTFM) berechnet und in der Maschine eingestellt werden.

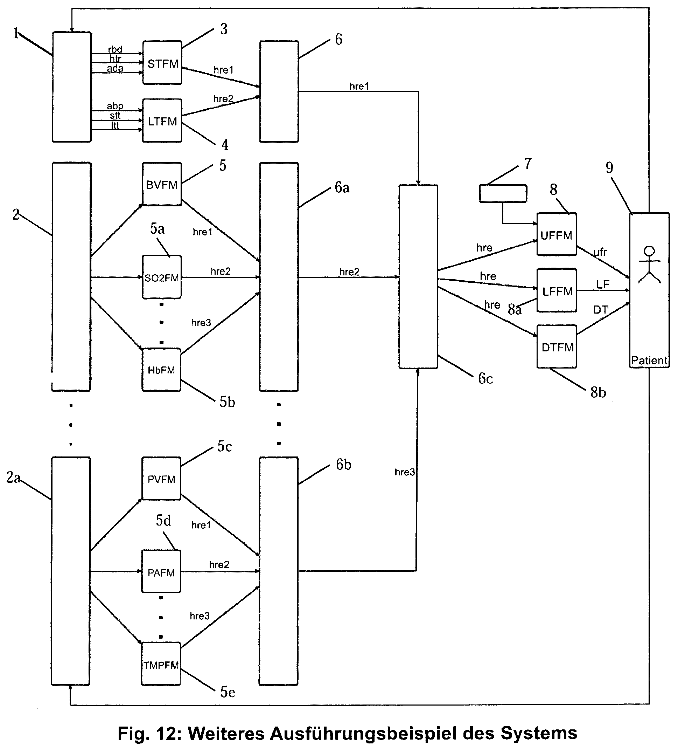

Eine mögliche Erweiterung des physiologischen Regelkreises ist in der Fig. 12 illustriert.

Die in Fig. 12 dargestellten Komponenten 1 bis 9 sind vorstehend bereits anhand der Fig. 1 erläutert. Die dortige Beschreibung trifft auch für das Ausführungsbeispiel gemäß Fig. 12 zu und wird folglich nicht erneut wiederholt.

Bei dem Ausführungsbeispiel gemäß Fig. 12 sind weitere Module vorgesehen, nämlich ein Sauerstoffsättigungs-Fuzzy-Modul 5a, ein Hämoglobin-Fuzzy-Modul 5b, ein Fuzzy-Modul 5c für den venösen Blutdruck, ein Fuzzy-Modul 5d für den arteriellen Blutdruck, und ein Fuzzy-Modul 5e für den transmembranen Druck. Die Fuzzy-Module 5a, 5b erhalten ihre Eingangsgrößen von der relativen Blutvolumenüberwachungseinrichtung 2. Die Fuzzy-Module 5c bis 5e erhalten ihre Eingangswerte von Maschinensensoren 2a, die den arteriellen Blutdruck, den venösen Blutdruck, den Transmembran-Druck usw. erfassen.

Das Ausführungsbeispiel gemäß Fig. 12 weist zusätzlich zu dem Gewichtungsmodul 6 weitere Gewichtungsmodule 6a, 6b und 6c auf. Das Gewichtungsmodul 6a führt hierbei eine Gewichtung von Eingangsgrößen hre1, hre2, hre3 durch, die von den Fuzzy-Modulen 5, 5a bzw. 5b ausgegeben werden.

Das Gewichtungsmodul 6b führt eine Gewichtung von Eingangsgrößen hre1, hre2, hre3 durch, die von den Fuzzy-Modulen 5c, 5d bzw. 5e generiert werden. Das Gewichtungsmodul 6c führt eine erneute Gewichtung der von den Gewichtungsmodulen 6, 6a, 6b jeweils ausgegebenen gewichteten Ausgangsgrößen hre1, hre2, hre3 durch und gibt ein oder mehrere gewichtete Ausgangssignale hre an das Fuzzy-Modul 8 bzw. an ein Dialysierflüssigkeitsleitfähigkeits-Fuzzy-Modul, LFFM, 8a und ein Dialysierflüssigkeitstemperatur-Fuzzy-Modul, DTFM, 8b ab. Entsprechend den gewichteten Ausgangssignalen hre und den von den Fuzzy-Modulen 8, 8a und 8b ausgegebenen Größen wird die Dialysierflüssigkeitsleitfähigkeit, die Dialysierflüssigkeitstemperatur und/oder die UF-Rate eingestellt.

Im Folgenden wird eine Simulation des Systems erläutert. Hierbei wurden einzelne Blöcke, die Module des in der Fig. 1 dargestellten physiologischen Regelkreises beinhalten, simuliert. Die dabei berechneten Profile der UF-Rate sind in den enstprechenden Figuren gezeigt. Die Daten, die zur Simulation verwendet wurden, sind reale Dialysepatientendaten. Das System zeigt in den UF-Profilen die Antwort der UF-Rate auf das Verhalten des Blutdrucks und des relativen Blutvolumens. Da in der Simulation kein Regelkreis realisiert werden kann, findet keine Antwort des Blutdrucks oder des relativen Blutvolumens auf die UF-Rate statt. Es wird nur eine Antwort der UF-Rate auf den BD- und den RBV-Verlauf gezeigt.

Folgende Blöcke des physiologischen Regelkreises beinhalten folgende Module:
Block Eins: STFM 3 und UFFM 8,
Block Zwei: LTFM 4 und UFFM 8,
Block Drei: BVFM 5 und UFFM 8,
Block Vier: STFM 3, LTFM 4, Gewichtungsmodul (Weighting Module) 6 und UFFM 8,
Block Fünf: STFM 3, LTFM 4, BVFM 5, Gewichtungsmodul (Weighting Module) 6 und UFFM 8.

Das Ablaufdiagramm der Blöcke Eins und Zwei ist in Fig. 13 dargestellt.

Bei dem in Fig. 13 dargestellten Ausführungsbeispiel eines im Rahmen der vorliegenden Erfindung liegenden Verfahrens ist die Funktionsweise der Blöcke Eins und Zwei dargelegt. In einem Schritt S10 erfolgt eine diskontinuierliche Blutdruckmessung, wobei die hierbei gewonnenen Werte in einer Speichereinheit S11 gespeichert und aus dieser dann auch wieder ausgelesen werden können. Bei einem Schritt S12 erfolgt eine Überprüfung, ob der gemessene Blutdruck den Grenzwert überschreitet oder nicht. Ist der Grenzwert überschritten worden, wird nachfolgend eine kontinuierliche Blutdruckmessung bei dem Schritt S13 durchgeführt, um eine genauere, schnellere Überwachung des Blutdrucks und des Blutdruckverlaufs zu erhalten.

Bei einem Schritt S14 wird der ermittelte Blutdruckverlauf evaluiert, also entweder der diskontinuierlich gemessene Blutdruck gemäß dem Verfahrensschritt S10, oder der kontinuierlich gemessene Blutdruck gemäß dem Verfahrensschritt S13 (bei einem den Grenzwert überschreitenden Blutdruck). In dem nachfolgenden Schritt S15 erfolgt eine Berechnung der Eingangsvariablen, die in einem Fuzzy-Inference-System in einem Schritt S16 verarbeitet werden. Zusätzlich kann das Ultrafiltrationsvolumen (UF-Volumen) in einem Schritt S18 ermittelt oder berücksichtigt werden.

In einer Steuerung oder Steuereinrichtung mit geschlossener Regelschleife wird bei einem Schritt S17 die jeweils passende Ultrafiltrationsrate ermittelt, die dann in einem Schritt S19 im Regelkreis als Stellgröße eingestellt wird. Der Patient 9 wird mit dieser Ultrafiltrationsrate behandelt, d.h. die Blutbehandlung wird so durchgeführt, dass die Ultrafiltrations-Sollrate erreicht oder zumindest angenähert wird.

Die Fig. 14 stellt eine Simulation des ersten Blocks dar und zeigt Simulationsergebnisse von Block Eins. Das STFM 3 bewertet Variablen, die den Blutdruck über höchstens die letzten 30 Minuten bewerten. Die BD-Werte bestehen aus getriggerten und aus berechneten BD-Werten. Betrachtet man die Fig. 13 im 30 Minuten Intervall, so zeigt der Blutdruck in diesen Intervallen keine Neigung zu einer Hypotension. Erst bei der Minute 220 erreicht der Blutdruck einen systolischen Wert von etwa 83 mmHg, was zu einer Reduktion der UF-Rate geführt hat.

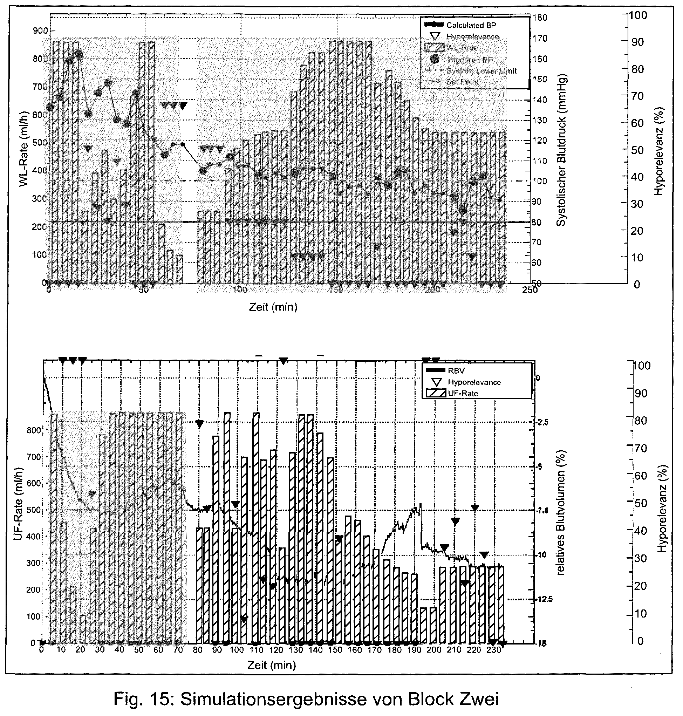

Die Fig. 15 stellt eine Simulation des zweiten Blocks, also der Module LTFM 4 und UFFM 8 dar, zeigt also Simulationsergebnisse vom Block Zwei. Das LTFM 4 berücksichtigt nur die getriggerten BD-Werte. Die Eingangsvariablen dieses Moduls beziehen BD-Werte ein, die innerhalb der letzten120 Minuten gemessen wurden. Wie die Fig. 15 zeigt, reagiert das zweite Modul sensibler auf BD-Änderungen als das STFM 3. Ein BD-Trend mit abfallendem Blutdruck kann rechtzeitig detektiert werden, was zu einer rechtzeitigen Reduktion der UF-Rate führt. Dies hat ein rechtzeitiges Feedback zur Folge.

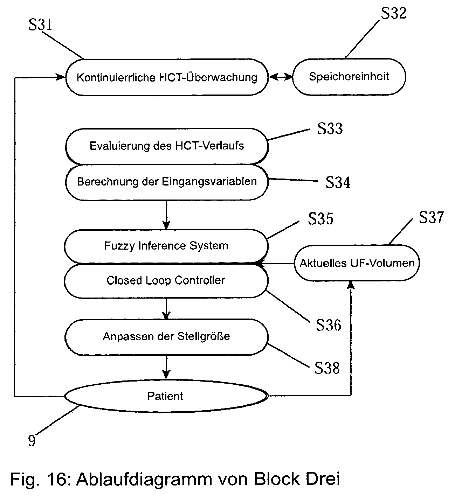

Das Ablaufdiagramm des Blocks Drei ist in der Abbildung gemäß Fig. 16 dargestellt.

In Fig. 16 ist ein Ablaufdiagramm, d.h. ein Ausführungsbeispiel eines Verfahrens dargestellt, das gemäß dem Block Drei durchgeführt wird. Bei einem Schritt S31 wird eine kontinuierliche Überwachung eines oder mehrerer Blutwerte durchgeführt. Beim Ausführungsbeispiel gemäß Fig. 16 wird der Hämatokritwert (HCT) kontinuierlich überwacht. Die bei der kontinuierlichen Blutwerteüberwachung des Schritts S31 ermittelten Messwerte werden in einer Speichereinheit S32 gespeichert, die bei einem oder mehreren Ausführungsbeispielen die gleiche Speichereinheit wie die Speichereinheit S11 gemäß Fig. 13 sein kann. Bei einem Schritt S33 wird der Blutwerteverlauf, insbesondere der Hämatokritverlauf evaluiert. Hieraus werden Eingangsvariable für ein Fuzzy-Modul berechnet, das beispielsweise eines der Fuzzy-Module 5, 5a bis 5e oder ein weiteres Fuzzy-Modul sein kann. Die in Schritt S34 berechneten Eingangsvariablen werden einem Fuzzy-Inferenz-System (Fuzzy Inference System) S35 zugeführt, das eine Steuerung mit geschlossener Regelschleife (Closed Loop Controller) S36 aufweisen kann. Als weitere Eingangsgröße erhält der Regler S36 oder das System S35 Informationen über das aktuelle Ultrafiltrationsvolumen, das bei einem Schritt S37 kontinuierlich oder intermittierend überwacht wird.

Der Regler S36 führt eine Anpassung der Stellgröße in einem Schritt S38 durch, mit der die Blutbehandlung des Patienten 9 durchgeführt wird, also die Ultrafiltrations-Sollrate angepasst wird.

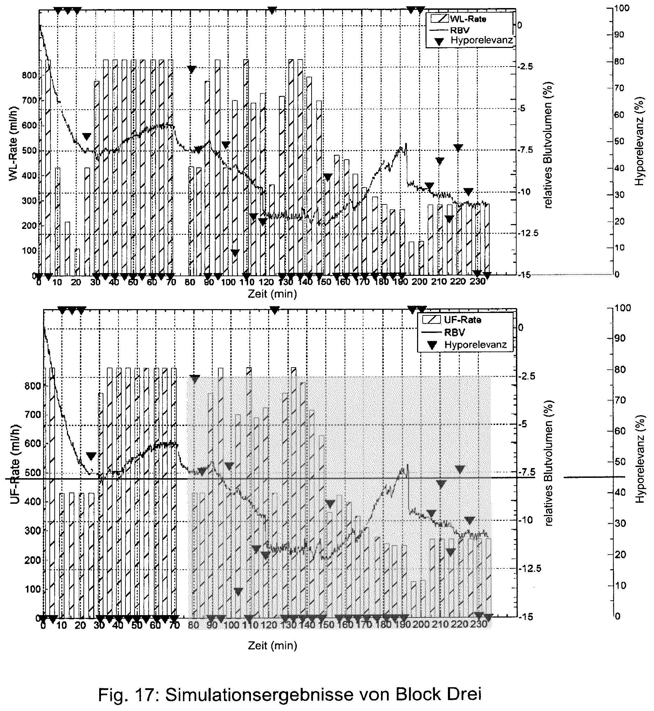

Fig. 17 zeigt Simulationsergebnisse von Block Drei. Das UF-Profil, das in der Fig. 17 gezeigt ist, soll zu einer Regelung des relativen Blutvolumens führen. Das RBV wird auf Steigungen in 10 Minuten-Intervallen bei einer 50 %-igen Überlappung geprüft. Je nach Stärke einer negativen Steigung wird die UF-Rate reduziert. Dadurch erreicht man eine Stabilisierung des relativen Blutvolumens, was zu einem stabileren Patientenzustand führen kann.

Es ist auch eine Simulation von STFM, LTFM und UFFM durchgeführt worden. Fig. 18 zeigt Simulationsergebnisse für die Module STFM 3 und LTFM 4. Fig. 18 stellt eine Simulation des vierten Blocks dar. In diesem Block ist das STFM 3 und das LTFM 4 implementiert. Jedes der Module berechnet eine Hyporelevanz, die in einer Gewichtungseinheit zu einer endgültigen Hyporelevanz kombiniert werden. Dadurch werden zwei wichtige Informationen geliefert: Die erste gibt das Verhalten des Kurzzeittrends des Blutdrucks inklusive der berechneten BD-Werte aus der GuideLine Technik an. Das STFM 3 liefert auch Informationen über den Abstand des letzten BD-Wertes zu einer voreingestellten BD-Sicherheitsgrenze, bei deren Überschreiten ein Alarm von der Maschine ausgelöst wird. Die zweite Information wird vom LTFM 4 geliefert und beinhaltet eine Bewertung des Blutdrucks über sein Langzeitverhalten. Diese beiden Informationen decken das Verhalten des Blutdrucks über 120 Minuten ab.

Die Reaktion der UF-Rate bei der Simulation des vierten Blocks ist ähnlich wie ihre Reaktion bei der Simulation des dritten Blocks. Ein markanter Unterschied tritt bei der Minute 215 auf, bei der eine UF-Raten-Reduktion aufgrund des STFMs 3 stattgefunden hat. Dies war in der Simulation des dritten Blocks nicht zu sehen.

Das Ablaufdiagramm des kompletten physiologischen Regelkreises ist in Fig. 19 dargestellt.

In Fig. 19 ist ein Ausführungsbeispiel des vollständigen, physiologischen Regelkreises gemäß einem Aspekt der Erfindung dargestellt. Die in Fig. 19 gezeigten Schritte und Merkmale S10 bis S15 entsprechen den anhand der Fig. 13 bereits erläuterten Schritten und Merkmalen S10 bis S15. Die Speichereinheit S11 kann hierbei zugleich als Speichereinheit S32 fungieren, die in Fig. 16 dargestellt ist.

Wie in Fig. 19 weiter dargestellt ist, enthält das Ausführungsbeispiel gemäß Fig. 19 auch die Schritte und Merkmale S31 bis S34 des Ausführungsbeispiels gemäß Fig. 16. Bei dem Ausführungsbeispiel gemäß Fig. 19 erfolgt somit sowohl eine Blutdruckmessung als auch eine Hämatokrit-Überwachung, wobei die Blutdruckmessung kontinuierlich oder diskontinuierlich durchgeführt werden kann. Ebenso kann die Hämatokrit-Überwachung kontinuierlich oder diskontinuierlich erfolgen. Bei dem in Fig. 19 dargestellten Ausführungsbeispiel wird von einer diskontinuierlichen Blutdruckmessung und einer kontinuierlichen Hämatokrit-Überwachung ausgegangen.

Beim Ausführungsbeispiel gemäß Fig. 19 ist eine Gewichtungseinheit S41 vorgesehen, die als Fuzzy-Modul ausgelegt sein kann, und die sowohl die im Schritt S15 berechneten Eingangsvariablen für den Blutdruckverlauf als auch die in dem Schritt S34 berechneten Eingangsvariablen für den Hämatokrit-Verlauf ermittelten Werte empfängt und in Abhängigkeit von internen, fest oder variabel vorgegebenen Gewichtungsfaktoren gewichtet. Die von der Gewichtungseinheit S41 abgegebene, auf der Basis des Blutdruck- und Hämatokritverlaufs gebildete Ausgangsgröße wird einem Fuzzy-Inferenz-System S42 zugeführt, das auch eine Steuereinrichtung bzw. einen mit geschlossener Regelschleife arbeitenden Regler S43 aufweist. Dem System S42 wird zudem als Eingangsgröße das ermittelte aktuelle Ultrafiltrationsvolumen, das im Schritt S44 ermittelt wird, zugeführt.

Der Regler S43 führt eine Stellgrößen-Anpassung S45 durch, mit der die Blutbehandlung des Patienten 9 ausgeführt wird.

Die Schritte bzw. Merkmale S42 bis S45 entsprechen im Wesentlichen den Schritten S16 bis S19 der Fig. 13 oder den Schritten S35 bis S38 der Fig. 16, mit der Besonderheit, dass die Eingangsgröße für das Fuzzy-Inferenz-System S16 bis S35 sowohl von dem Blutdruckverlauf als auch von dem Hämatokritverlauf abhängt und diese beiden Parameter gewichtet in das Eingangssignal des Fuzzy-Inferenz-Systems S16 bzw. S35 eingehen.

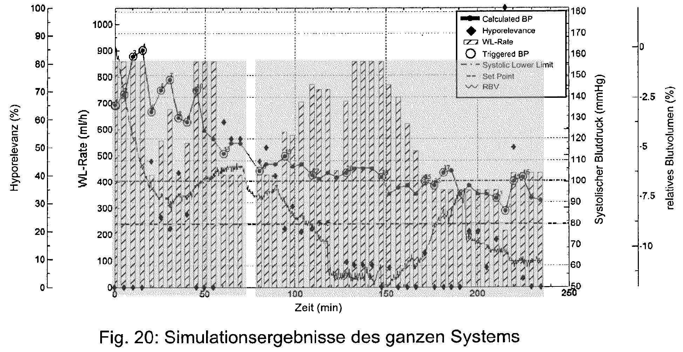

Es wurde eine Simulation von STFM 3, LTFM 4, BVFM 5 und UFFM 8 durchgeführt. Fig. 20 simuliert den kompletten physiologischen Regelkreis. Fig. 20 zeigt Simulationsergebnisse des ganzen Systems. Das UF-Raten-Profil stellt eine Kombination aus allen drei Eingangsmodulen dar. Jedes Modul berechnet unabhängig von den anderen Modulen eine Hyporelevanz, die durch eine Gewichtungseinheit zu einer endgültigen Hyporelevanz kombiniert wird. Durch diese Gewichtung erreicht man eine von den Gewichtungen abhängige Steuerung der Stellgröße, was zu einer von den Gewichtungen abhängigen Regelung der Eingangsgrößen führt.

Bei einem, mehreren oder allen Ausführungsbeispielen ist somit ein neuartiger physiologischer Regelkreis realisiert, der mindestens zwei Regelparameter beinhaltet, nämlich den Blutdruck und das relative Blutvolumen, die in drei oder mehr unterschiedlichen und unabhängigen Fuzzy-Modulen (STFM 3, LTFM 4 und BVFM 5) verarbeitet werden. Eine Gewichtungseinheit 6 kombiniert die aus den drei oder mehr Fuzzy-Modulen entstandenen Werte, die den Zustand des Patienten mit einem Wert zwischen 0 und 100 % beschreiben, zu einer endgültigen Aussage über seinen Zustand. Diese wird zusammen mit dem relativen UF-Volumen, das das Verhältnis aus dem aktuellen und dem totalen UF-Volumen ist, in einem weiteren Fuzzy-Modul zu einer Berechnung der UF-Rate des Patienten verarbeitet.

### ABKÜRZUNGEN

- ada: Adaptation
- BD: Blutdruck
- bpd: blood pressure difference, Blutdruckdifferenz
- BVFM: Blutvolumen-Fuzzy-Modul
- DT: Dialysierflüssigkeitstemperatur
- DTFM: Dialysierflüssigkeitstemperatur-Fuzzy-Modul
- HCT: Hämatokrit
- Hb: Hämoglobin
- hre: Hyporelevanz
- htr: Hypotonietrend
- LF: Dialysierflüssigkeitsleitfähigkeit
- IIs: linear least square
- LFFM: Dialysierflüssigkeitsleitfähigkeits-Fuzzy-Modul
- LTFM: Langzeit (Long-Time) - Fuzzy-Modul
- Itt: long time trend, Langzeittrend
- MIMO: Multi Input Multi Output
- nibp: nichtinvasiver Blutdruck
- PA: Arterieller Druck
- PAFM: Arterieller Druck - Fuzzy-Modul
- PV: Venöser Druck
- PVFM: Venöser Druck - Fuzzy-Modul
- rbd: relative blood pressure decrease, relative Blutdruckabnahme
- RBV: Relatives Blutvolumen
- SO2: Sauerstoffsättigung
- STFM: Kurzzeit (Short Time) - Fuzzy Modul
- stt: short time trend, Kurzzeittrend
- SVR: Support Vector Regression
- TMP: Transmembrandruck
- TMPFM: Transmembrandruck-Fuzzy-Modul
- Trigg: getriggert (triggered)
- UF: Ultrafiltration
- WLR: Gewichtsverlustrate, Weight Loss Rate

## Patentansprüche

1. System zur Blutbehandlung,
welches dazu ausgelegt ist, mindestens zwei hämodynamische Parameter, zumindest Blutdruck und relatives Blutvolumen, während der Blutbehandlung zu erfassen, wobei mindestens zwei Fuzzy-Module (3 bis 5, 5a-5e) vorhanden sind und mit mindestens einem Gewichtungsmodul (6, 6a, 6b, 6c), dem mindestens zwei der von den Fuzzy-Modulen (3 bis 5, 5a-5e) abgegebenen Ausgangssignale zuführbar sind, und mit mindestens einer Einstelleinrichtung (8, 8a, 8b) zur Einstellung mindestens einer Stellgröße, in Abhängigkeit von einem von dem Gewichtungsmodul (6, 6a, 6b, 6c) abgegebenen Ausgangssignal, **dadurch gekennzeichnet, dass** die mindestens zwei Fuzzymodule (3 - 5, 5a - 5e) als Eingangsgrößen Messwerte der hämodynamischen Parameter aufnehmen und dass das System modular ausbaubar ist, sodass weitere Fuzzymodule (3 - 5, 5a - 5e) hinzugefügt werden können, um eine Einbeziehung weiterer hämodynamischer Parameter zu ermöglichen.

2. System nach Anspruch 1, das zur Erfassung oder Vermeidung von intradialytischen hypotensiven Episoden ausgelegt ist, und bei dem die überwachten hämodynamischen Parameter zwei oder mehr der folgenden Größen umfassen:
Blutdruck, Blutdruckverlauf, relatives Blutvolumen, relativer Blutvolumenverlauf, Hämatokritwert, Hämatokritverlauf,Sauerstoffsättigung, Sauerstoffsättigungsverlauf des Bluts, Herzrate, Herzratenverlauf Absorbanz urämischer Toxine oder ähnliche hämodynamische Parameter oder hämodynamische Verläufe, oder physikalische Parameter oder physikalische Verläufe der vom System gemessenen Blutdruckwerte oder arterielle und/oder venöse Blutdruckverläufe.

3. System nach Anspruch 1 oder 2, das als Dialysegerät für eine Hämodialyse, Hämofiltration oder Hämodiafiltration ausgelegt ist.

4. System nach einem der vorhergehenden Ansprüche, das dazu ausgelegt ist, die Blutdruckwerte diskontinuierlich in bestimmten Zeitintervallen zu messen, mit einem Grenzwert zu vergleichen und bei Abfallen des Blutdruckwerts unter den Grenzwert auf kontinuierliche Blutdruckmessung überzugehen.

5. System nach einem der vorhergehenden Ansprüche, bei dem ein Kurzzeit-Fuzzy-Modul (3) vorhanden ist, das dazu ausgelegt ist, das Verhalten des Blutdrucks in einem zurückliegenden Zeitraum zu bewerten und anhand von Evaluierungsregeln eine den Zustand des Patienten (9) widerspiegelnde Variable zu berechnen.

6. System nach Anspruch 5, bei dem ein Langzeit-Fuzzy-Modul (4) vorgesehen ist, durch das, nach einer Kurvenanpassung mittels eines Algorithmus, der Verlauf des Blutdrucks über einen zurückliegenden Zeitraum ausgewertet wird, der länger ist als der von dem in Anspruch 5 genannten Fuzzy-Modul (3) (Kurzzeit-Fuzzy-Modul) ausgewertete Zeitraum.

7. System nach einem der vorhergehenden Ansprüche, mit einer Messeinheit in Form eines Hämosensors (2a), z.B. eines Hämatokritsensors, Hämoglobin-Sensors oder Sauerstoffsättigungssensors, und einem weiteren Fuzzy-Modul (5), das zur Auswertung des von dem Hämosensor (2a) abgegebenen Messsignals ausgelegt ist und das das Blutvolumen sowie den Blutvolumenverlauf eines Patienten auswertet.

8. System nach Anspruch 2 bis 7, bei dem der relative Blutvolumenverlauf, RBV, in Form einer RBV-Kurve gespeichert wird, die RBV-Kurve mit unterschiedlichen Fenstergrößen mit einem Algorithmus angenähert wird, und der Blutvolumenverlauf in Zeitabständen überwacht wird, die einer Fenstergröße entsprechen, bei der sich eine ausreichend gute Überlappung von beispielsweise 50% oder mehr in dem tatsächlichen Blutvolumenverlauf ergibt.

9. System nach einem der vorhergehenden Ansprüche, bei dem mindestens zwei oder drei der Fuzzy-Module (3) jeweils einen eine Hyporelevanz darstellenden Ausgangswert (hre1, hre2, hre3) bilden, wobei das Gewichtungsmodul (6, 6a, 6b, 6c) dazu ausgelegt ist, die von den Fuzzy-Modulen (3, 4, 5) gebildeten Hyporelevanz-Ausgangswerte zu einem resultierenden Hyporelevanz-Ausgangswert zu kombinieren, und mit einem weiteren Fuzzy-Modul, (8) das den resultierenden Hyporelevanz-Ausgangswert zusammen mit einem relativen Ultrafiltrationsvolumen, das das Verhältnis zwischen dem aktuellen und dem gesamten Ultrafiltrationsvolumen beschreibt, oder die relative Zeit, die das Verhältnis aus der aktuellen und der gesamten Zeit beschreibt, evaluiert, und eine entsprechende Ultrafiltrations-Sollrate berechnet.

10. System nach einem der vorhergehenden Ansprüche, wobei die Stellgrösse eine Ultrafiltrationsrate ist.

11. System nach einem der vorhergehenden Ansprüche, wobei die Stellgrösse eine Dialysierflüssigkeitstemperatur ist.

12. System nach einem der vorhergehenden Ansprüche, wobei die Stellgrösse eine Dialysierflüssigkeitsleitfähigkeit ist.

## Claims

1. A system for blood treatment which is configured to detect at least two hemodynamic parameters, at least blood pressure and relative blood volume, during the blood treatment, wherein at least two fuzzy modules (3 to 5, 5a-5e) are provided and comprising at least one weighting module (6, 6a, 6b, 6c) to which at least two of the output signals transmitted by the fuzzy modules (3 to 5, 5a-5e) can be supplied, and comprising at least one setting means (8, 8a, 8b) for setting at least one variable in response to an output signal transmitted by the weighting module (6, 6a, 6b, 6c), **characterized in that** the at least two fuzzy modules (3 to 5, 5a-5e) receive measured values of the hemodynamic parameters as input variables and that the system can be modularly extended, so that further fuzzy modules (3 to 5, 5a-5e) can be added to enable incorporation of further hemodynamic parameters.

2. The system according to claim 1, which is configured for detecting or avoiding intradialytic hypotensive episodes and in which the hemodynamic parameters monitored comprise two or more of the following parameters:
blood pressure, course of blood pressure, relative blood volume, course of relative blood volume, hematocrit value, hematocrit course, oxygen saturation, course of oxygen saturation of the blood, heart rate, course of heart rate, absorbance of uremic toxins or similar hemodynamic parameters or hemodynamic courses, or physical parameters or physical courses of blood pressure values measured by the system or course of arterial and/or venous blood pressure.

3. The system according to claim 1 or 2, which is designed as a dialyzer for hemodialysis, hemofiltration or hemodiafiltration.

4. The system according to any one of the preceding claims, which is configured to discontinuously measure the blood pressure values at predetermined time intervals, to compare them to a limit and, in the case of decrease of the blood pressure value below the limit, to change to continuous blood pressure measurement.

5. The system according to any one of the preceding claims, in which a short time fuzzy module (3) is provided which is configured to evaluate the behavior of the blood pressure in an earlier period of time and to compute a variable reflecting the patient's (9) condition by way of evaluating rules.

6. The system according to claim 5, in which a long time fuzzy module (4) is provided by which the course of the blood pressure is evaluated after curve adaptation by means of an algorithm over an earlier period of time which is longer than the period of time evaluated by the fuzzy module (3) (short time fuzzy module) mentioned in claim 5.

7. The system according to any one of the preceding claims, comprising a measuring unit in the form of a hemosensor (2a), e.g. a hematocrit sensor, hemoglobin sensor or oxygen saturation sensor, and a further fuzzy module (5) configured to evaluate the measuring signal transmitted by the hemosensor (2a) and evaluating the blood volume and the course of the blood volume of a patient.

8. The system according to one of the claims 2 to 7, in which the course of the relative blood volume RBV is stored in the form of a RBV curve, the RBV curve with different window sizes is approached by an algorithm, and the course of the blood volume is monitored at time intervals corresponding to a window size in which sufficient overlapping of, e.g., 50% or more is resulting in the actual course of the blood volume.

9. The system according to any one of the preceding claims, in which at least two or three of the fuzzy modules (3) form a respective output value (hre1, hre2, hre3) representing a hyporelevance, wherein the weighting module (6, 6a, 6b, 6c) is configured to combine the hyporelevance output values formed by the fuzzy modules (3, 4, 5) into a resulting hyporelevance output value, and comprising a further fuzzy module (8) that evaluates the resulting hyporelevance output value together with a relative ultrafiltration volume, which describes the ratio between the current and the total ultrafiltration volume, or evaluates the relative time, which describes the ratio of the current and the total time, and computes a corresponding desired ultrafiltration rate.

10. The system according to any one of the preceding claims, in which the variable is an ultrafiltration rate.

11. The system according to any one of the preceding claims, in which the variable is a dialysis fluid temperature.

12. The system according to any one of the preceding claims, in which the variable is a dialysis fluid conductivity.

## Revendications

1. Système pour le traitement du sang,
qui est conçu afin de détecter au moins deux paramètres hémodynamiques, au moins la tension artérielle et le volume sanguin relatif, pendant le traitement du sang, au moins deux modules de logique floue (3 à 5, 5a-5e) étant présents et avec au moins un module de pondération (6, 6a, 6b, 6c), auquel au moins deux des signaux de départ émis par les modules de logique floue (3 à 5, 5a-5e) peuvent être transmis, et avec au moins un dispositif de réglage (8, 8a, 8b) pour le réglage d'au moins une grandeur de réglage en fonction d'un signal de départ émis par le module de pondération (6, 6a, 6b, 6c), **caractérisé en ce que** les au moins deux modules de logique floue (3-5, 5a-5e) reçoivent, comme grandeurs d'entrée, des valeurs de mesure des paramètres hémodynamiques et **en ce que** le système est extensible de manière modulaire de sorte que d'autres modules de logique floue (3-5, 5a-5e) puissent être ajoutés afin de permettre une intégration d'autres paramètres hémodynamiques.

2. Système selon la revendication 1, qui est conçu pour la détection ou l'évitement d'épisodes hypotensifs intradialytiques et pour lequel les paramètres hémodynamiques surveillés comportent deux des grandeurs suivantes ou plus :
tension artérielle, courbe de tension artérielle, volume sanguin relatif, courbe de volume sanguin, valeur d'hématocrite, courbe d'hématocrite, saturation en oxygène, courbe de saturation en oxygène du sang, fréquence cardiaque, courbe de fréquence cardiaque, absorption de toxines urémiques ou paramètres hémodynamiques similaires ou courbes hémodynamiques, ou paramètres physiques ou courbes physiques des valeurs de tension artérielle mesurée par le système ou courbes de tension artérielles et/ou veineuses.

3. Système selon la revendication 1 ou 2 qui est conçu comme appareil de dialyse pour une hémodialyse, hémofiltration ou hémodiafiltration.

4. Système selon l'une quelconque des revendications précédentes, qui est conçu afin de mesurer des valeurs de tension artérielle en discontinu dans des intervalles de temps déterminés, de les comparer avec une valeur limite et de passer, en cas de chute de la valeur de tension artérielle sous la valeur limite, à une mesure de tension artérielle continue.

5. Système selon l'une quelconque des revendications précédentes, pour lequel un module de logique floue à court terme (3) est présent, lequel est conçu afin d'évaluer le comportement de la tension artérielle dans une période précédente et de calculer à l'aide de règles d'évaluation une variable reflétant l'état du patient (9).

6. Système selon la revendication 5, pour lequel un module de logique floue à long terme (4) est prévu, par lequel après une adaptation de courbe à l'aide d'un algorithme, la courbe de la tension artérielle est évaluée dans une période précédente qui est plus longue que la période évaluée par le module de logique floue (3) cité à la revendication 5 (module de logique floue à court terme).

7. Système selon l'une quelconque des revendications précédentes, avec une unité de mesure sous la forme d'un hémocapteur (2a), par exemple d'un capteur d'hématocrite, capteur d'hémoglobine ou capteur de saturation en oxygène, et un autre module de logique floue (5) qui est conçu pour l'évaluation du signal de mesure émis par l'hémocapteur et qui évalue le volume sanguin ainsi que la courbe de volume sanguin d'un patient.

8. Système selon la revendication 2 à 7, pour lequel la courbe de volume sanguin relatif, RBV, est enregistrée sous la forme d'une courbe RVB qui est approchée avec différentes grandeurs de fenêtre avec un algorithme, et la courbe de volume sanguin est surveillée à des intervalles de temps qui correspondent à une grandeur de fenêtre, pour laquelle un chevauchement suffisamment bon de par exemple 50 % ou plus se produit dans la courbe de volume sanguin réelle.

9. Système selon l'une quelconque des revendications précédentes, pour lequel au moins deux ou trois des modules de logique floue (3) forment respectivement une valeur de départ (hrel, hre2, hre3) représentant une hypopertinence, le module de pondération (6, 6a, 6b, 6c) étant conçu afin de combiner les valeurs de départ d'hypopertinence formées par les modules de logique floue (3, 4, 5) en une valeur de départ d'hypopertinence résultante et avec un autre module de logique floue (8) qui décrit la valeur de départ d'hypopertinence résultante conjointement avec un volume d'ultrafiltration relatif qui décrit le rapport entre le volume d'ultrafiltration actuel et le volume d'ultrafiltration entier, ou évalue le temps relatif qui décrit le rapport entre le temps actuel et le temps entier et calcule une vitesse de consigne d'ultrafiltration correspondante.

10. Système selon l'une quelconque des revendications précédentes, la grandeur de réglage étant une vitesse d'ultrafiltration.

11. Système selon l'une quelconque des revendications précédentes, la grandeur de réglage étant une température de liquide de dialyse.

12. Système selon l'une quelconque des revendications précédentes, la grandeur de réglage étant une conductivité de liquide de dialyse.
